# EUROPEAN PATENT SPECIFICATION

(11) **EP 3 920 812 B1**
(45) Date of publication and mention of the grant of the patent: **18.03.2026**
(21) Application number: 20712082.5
(22) Date of filing: 07.02.2020
(51) Int. Cl.: A61B 17/16, A61B 17/32

(54) **ROTARY OSCILLATING AND RECIPROCATING SURGICAL TOOL**
ROTIERENDES OSZILLIERENDES UND HIN- UND HERGEHENDES CHIRURGISCHES WERKZEUG
OUTIL CHIRURGICAL ROTATIF OSCILLANT ET ANIMÉ D'UN MOUVEMENT DE VA-ET-VIENT

(30) Priority: 08.02.2019 US 201962803039 P
(43) Date of publication of application: 15.12.2021
(73) Proprietor: Globus Medical, Inc., Audubon, PA 19403 (US)
(72) Inventor: LARK, James, D., Bloomfield, MI 48324 (US); SCALES, John, S., Ann Arbor, MI 48105 (US); BONO, Peter, L., Bingham Farms, MI 48025 (US)
(74) Representative: Morabito, Sara
(86) International application number: PCT/US2020/017127
(87) International publication number: WO 2020/163667

(56) References cited:
- WO-A2-2019/083983
- US-B2- 10 194 922

## Description

### FIELD OF THE INVENTION

The present invention relates to a powered surgical tool with a cutter adapted to modify tissue such as bone, cartilage and discs. The tool effects both rotary oscillation and longitudinal reciprocation of the cutter.

### BACKGROUND OF THE INVENTION

The prior art has provided surgical tools having a rotary cutter adapted to modify tissue such as bone, cartilage and discs in a patient. Such tools, though, present a problem if the cutter encounters fibrous tissue such as muscle and nerves. Such fibrous tissue can wrap around the cutter and be damaged thereby. The prior art has also provided oscillating rotary tools for such surgical procedures, but the mechanisms used to effect oscillation of the cutter during its rotation do not operate smoothly due to the mechanisms used to effect oscillation. An advance in such oscillating tools is represented by our co-pending applications: U.S. Non-Provisional Patent Application No. 13/469,665, entitled "Rotary Oscillating Bone, Cartilage, and Disk Removal Tool Assembly, filed May 11, 2012; and now issued Patent No. 10,194,922, issued on February 5, 2019; U.S. International Application No. PCT/US2013/037071, entitled "Rotary Oscillating Bone, Cartilage, and Disk Removal Tool Assembly", filed April 18, 2013; U.S. Non-Provisional Patent Application 13/647,101, entitled "Cutting Tool for Bone, Cartilage, and Disk Removal", filed October 8, 2012, and now issued Patent No. 9,232,953, issued on January 12, 2016; U.S. International Application No. PCT/US2013/063182, entitled "Cutting Tool for Bone, Cartilage, and Disk Removal", filed October 3, 2013; U.S. Provisional Patent Application No. 62/460,481, entitled "Surgical Rotary Tool", filed February 17, 2017, U.S. Non-Provisional Patent Application 15/895,352, entitled "Surgical Rotary Tool", filed February 13, 2018; and U.S. Non-Provisional Patent Application 15/932,361, entitled "Surgical Rotary Tool", filed February 16, 2018; U.S. Provisional Patent Application No. 62/423,624, entitled "Rotary Oscillating Surgical Tool", filed November 17, 2016, and US Non-Provisional Patent Application 15/814,891, entitled "Rotary Oscillating Surgical Tool", filed November 16, 2017; U.S. Provisional Patent Application No. 62/423,651, entitled "Robotic Surgical System", filed November 17, 2016; U.S. Provisional Patent Application No. 62/423,677, entitled "Robotic Surgical System", filed November 17, 2016, and U.S. Non-Provisional Patent Application 15/816,861, entitled "Robotic Surgical System", filed November 17, 2017.

Such tools are typically small and lightweight, with little room for drive mechanisms. They tend to operate at high cutting speeds for cutting efficiency and control by a surgeon. Oscillations are on the order of at least about 10,000 oscillations per minute (5,000 orbits per minute), and may be 30,000-50,000 oscillations per minute or more. Reciprocation rate is preferably the same. An oscillation is movement of the cutter from one rotational position extreme to its other rotational extreme. Reciprocation is movement of the cutter from one linear movement position extreme to its other linear movement extreme. The cutter configuration and material being removed will determine cutter speed. Because of the high speed and need for precision placement and cutting, the tools need to be smooth in operation with little vibration.

WO2019/083983 describes surgical tools known in the art.

### SUMMARY OF THE INVENTION

According to the invention it is provided a surgical tool having the features outlined in claim 1. Further advantageous aspects of the invention are set forth in the dependent claims.

According to one embodiment of the present invention, a surgical tool is provided with a housing, a cutter support shaft that is operably connected to a motor to effect oscillating rotation of the shaft, and a drive transmission configured between the motor and the shaft to effect oscillating rotary movement and simultaneous linear reciprocating movement of the shaft and cutter mounted to the shaft.

It is an objective of the instant invention to provide an oscillation/reciprocation effecting drive transmission that utilizes a first driver to effect rotary oscillation of a cutter and to simultaneously effect driving of a second driver that is operable to add longitudinal reciprocating movement to the cutter.

It is yet another objective of the instant invention to provide an oscillation/reciprocation effecting drive transmission that utilizes a rack and pinion gear arrangement to effect driving connection between the first and second drivers. It is a still further objective of the instant invention to provide a reciprocation effecting driver coupled to the oscillation driver to effect simultaneous longitudinal reciprocation of the cutter shaft while it oscillates.

It is yet another objective of the instant invention to provide a drive transmission that is simple in construction.

Other objects and advantages of this invention will become apparent from the following description taken in conjunction with any accompanying drawings wherein are set forth, by way of illustration and example, certain embodiments of this invention. Any drawings contained herein constitute a part of this specification, include exemplary embodiments of the present invention, and illustrate various objects and features thereof.

### BRIEF DESCRIPTION OF THE FIGURES

The invention is shown in figures 19-21, the remaining figures are reported for a better understanding of the invention.
Fig. 1 is a perspective view of the surgical tissue removal tool;
Fig. 2 is a cutaway fragmentary perspective view of the surgical tool of Fig. 1;
Fig. 3 is an exploded perspective view showing details of the internal parts of the surgical tool shown in Fig. 1;
Fig. 4 is a top plan view of the details of the internal parts of the surgical tool shown in Fig. 1;
Fig. 5 is a fragmentary side view showing two drivers in the surgical tool shown in Fig. 1;
Figs. 6A-6C illustrate various rotary positions of components of the second driver in the surgical tool that effect reciprocating movement of the cutting tool;
Figs. 7A1-7A9 illustrate a Cardan type first driver of the transmission;
Figs. 7B1-7B4 illustrate the Cardan drive of Fig. 7A, and also include the output of the first driver and the input of the second driver;
Fig. 8 is a fragmentary perspective view of one embodiment of a drive transmission;
Fig. 9 is a fragmentary side elevation view of a drive transmission as seen in Fig. 5;
Fig. 10 is a fragmentary perspective view of a drive transmission similar to that shown in Fig. 8;
Fig. 11 is a fragmentary perspective view of another embodiment of a drive transmission similar to that shown in Fig. 8, but with an alternate second driver;
Fig. 12 is a fragmentary perspective view of a drive transmission showing details of parts in Fig. 11;
Fig. 13 is a fragmentary perspective view of another embodiment of a drive transmission;
Fig. 14 is an exploded fragmentary perspective view of a portion of the drive transmission shown in Fig. 13;
Fig. 15 is a fragmentary perspective view of portions of the drive transmission of Fig. 13;
Fig. 16 is a fragmentary perspective view of portions of the drive transmission of Fig. 13;
Fig. 17 is a fragmentary perspective view of portions of a still further embodiment of a drive transmission;
Figs. 18A-18D are fragmentary perspective views of portions of the transmission of Fig. 17 showing sequential positions of portions of the second drive effecting reciprocating movement of a cutter shaft and associated cutter;
Fig. 19 shows a fragmentary perspective view of another embodiment of a drive transmission with a drive rack in a first rotated position;
Fig. 20 shows a fragmentary perspective view of the drive transmission with a drive rack in a second rotated position; and
Fig. 21 is a side elevation view of the drive transmission of Figs. 19, 20 with sections broken away to show details thereof.

### DETAILED DESCRIPTION OF THE INVENTION

The reference numeral 30 designates, generally, a rotary oscillating and reciprocating surgical tool useful, particularly, in the modification and/or removal of hard tissue such as bone, cartilage and disc. The surgical tool 30 is a handheld tool with a housing 32 providing a handle 34 for manually gripping the tool 30 for use during a surgical procedure. While one shape and style of handle 34 is illustrated, any suitable shape and style of handle can be provided. For example, a right angle pistol grip may be added. Additionally, the housing may have a narrow front portion for a smaller pencil-like "precision grip", while the larger remaining portion is sized to balance in the user's hand, such as in the web area between the index finger and thumb, for allowing better control with less fatigue.

The tool 30 can be used in surgical operations such as spinal surgery, wherein tissue such as bone, cartilage and disc material that is preferably of a non-fibrous tissue type may be modified or removed, such as from the spine of a patient. The tool 30 has an output shaft 36, which is driven to rotate in an oscillating manner of two alternate directions about the longitudinal axis of the shaft 36 by a drive transmission 35 that has two drive components, including an oscillation effecting first driver 37. Shaft 36 is provided with a cutting tool 38 positioned and secured to a distal end portion of the shaft 36. The cutting tool 38 is driven to rotate in alternate directions (oscillation) like the shaft 36, with a limited range of angular displacement of rotation, for example, between about 90° and about 180°. It has been found that such oscillatory rotation is effective in cutting or modifying hard tissue like bone, cartilage and portions of discs. It has also been found that this oscillatory rotation reduces the risk of damage to fibrous tissue such as muscle and nerve. The tool 30 is provided with the transmission 35 that includes the driver 37 to effect the oscillating rotation of the shaft 36 and its attached cutting tool 38. The transmission 35 is preferably provided with a reciprocation effecting second driver 39 coupled to the first driver 37 to simultaneously effect reciprocating motion of the shaft 36 and cutting tool 38 while they are oscillating. The second driver 39 uses the oscillating output of the first driver 37 to add the reciprocating motion to the shaft 36 and cutting tool 38. Reciprocating movement is parallel to the longitudinal axis of the shaft 36. The first driver 37 is upstream operationally of the second driver 39.

The tool 30 can receive energy for its operations from an external supply, such as a direct current power supply cord 40. A power control switch 42 can be provided on the housing 32 for controlling the operation of the tool 30, such as in an ON and OFF manner and/or in a variable speed manner. A light source 44 may also be provided on the housing 32 for illuminating the surgical site. Such a light source may be a light emitting diode (LED), which can be powered directly or indirectly by energy from cord 40. Energy can also be provided by a battery 46 or other energy storage device.

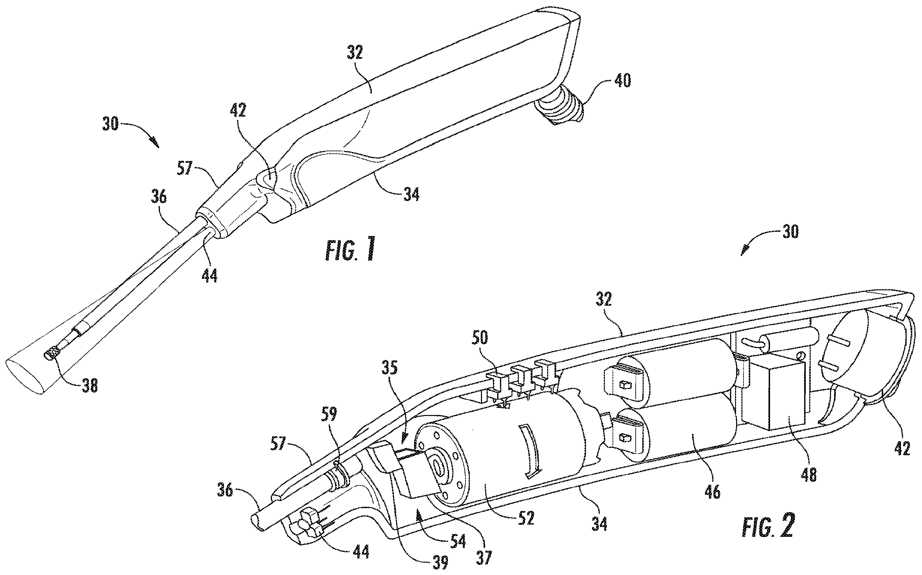

Fig. 2 illustrates internal components of the tool 30. An energy source can be provided by a battery supply 46 mounted in the housing 32. The battery supply 46 can be charged by the power cord 40. Electronics 48 are provided in the housing 32 for controlling the operation of the tool 30. A plurality of indicator lamps 50 may also be provided on the housing 32 and can be LEDs for indicating operational characteristics of the tool 30, such as the state of charge of the battery supply 46. Alternately, the batteries 46 can be eliminated in favor of the cord 40 being connected to a source of electrical energy. Preferably, the power supply is low voltage, e.g., 12 volts. Additionally, the motor 52 can be powered by compressed air, a vacuum, or any other suitable source of energy that would, on demand, effect rotation of a rotor portion of the motor 52.

The motor 52 is suitably mounted in the housing 32, wherein a portion of the motor, a rotor (not shown), is free to rotate and ultimately drive the shaft 36. A portion of the motor 52 is fixed against rotation in the housing 32 as is known in the art; for example, a motor housing and/or stator. The motor 52 drives the shaft 36 through the transmission 35 and its drivers 37, 39. The first driver 37 is operable for converting continuous rotary motion from the motor 52 to rotary oscillation of the shaft 36. The second driver 39 is operable for converting continuous oscillation from the first driver 37 and continuous rotation of the motor 52, and adds continuous reciprocating longitudinal movement to the shaft 36. The shaft 36 is suitably mounted in the nose 57 of the housing 32, as in one or more bearings 59. Operationally, the first driver 37 is upstream of the second driver 39. The journal bearings 59 need to accomodate both rotary and linear movement of the shaft 36, and a suitable bearing is a journal bearing. The shaft 36 may be angled relative to the longitudinal axis of the housing 32, as depicted in Fig. 1, for ergonomics. Cooling fins, or a cooling fan, (not shown) may be attached to or near the motor 52 for cooling the motor and/or the tool 30.

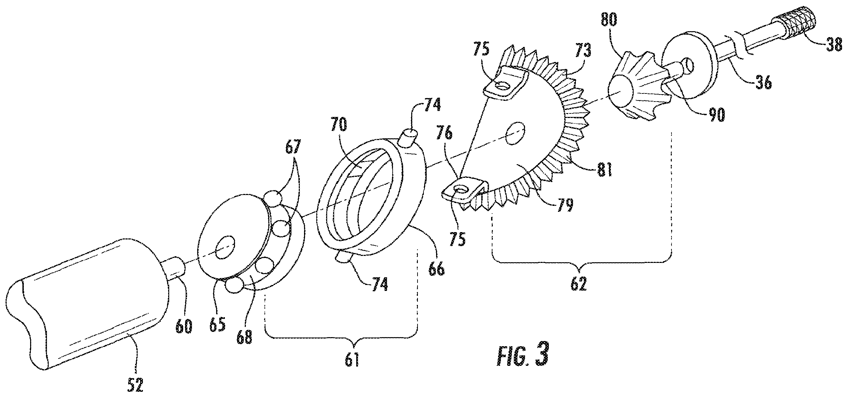

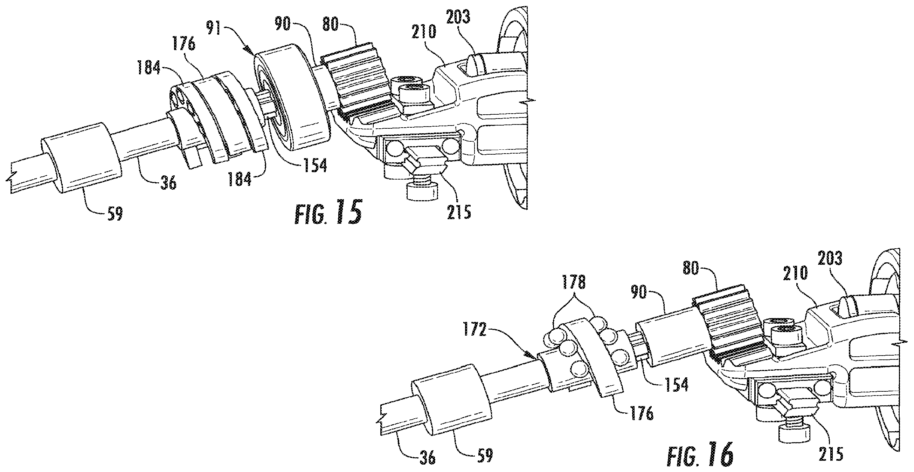

Figs. 3-18 illustrate different forms of drivers 37 and 39.

The first driver 37, as best seen in Figs. 3-4, is positioned in the housing 32 and operably couples the second driver 39, and hence shaft 36, to the motor 52, and is operable to convert the continuous rotary motion of the shaft 60 of the motor 52 to oscillating rotary motion of the shaft 36. By oscillating rotary motion, it is meant that the shaft 36 will rotate a portion of a complete revolution first in one rotation direction and then in the other rotation direction, first counterclockwise, then clockwise, then counterclockwise again and so on. To effect this movement, the transmission 35 comprises the two driver components 37, 39. The first driver 37 is operable to convert the rotary motion of the shaft 60 of the motor 52 to oscillating rotary motion of the shaft 36, and the second driver 39 is operable to convert that oscillating motion to reciprocating linear motion while maintaining the oscillating motion.

In the illustrated embodiment, the first transmission driver 37 includes a ball bearing having an inner race 65, an outer race 66 and a plurality of bearing balls 67 contained in the races 65, 66. The inner race 65 is secured to the motor shaft 60 for rotation thereby about the central axis of the motor shaft 60. In the illustrated embodiment, the inner race 65 is in the form of a sphere, with a groove 68 therein, and sized to receive and retain the balls 67 therein. The outer race 66 is in the form of a ring, having a groove 70 recessed in the inner surface thereof, and sized to receive and retain the balls 67 therein. The grooves 68, 70 open toward one another and are positioned in a plane P that is set at an angle A relative to the longitudinal axis of the motor shaft 60. The angle A is the smallest angle between the plane P and shaft axis since the angle of the plane P relative to the shaft axis changes depending on the position from which the measurement is taken. The angle A is in the range of between about 30° and about 80°.

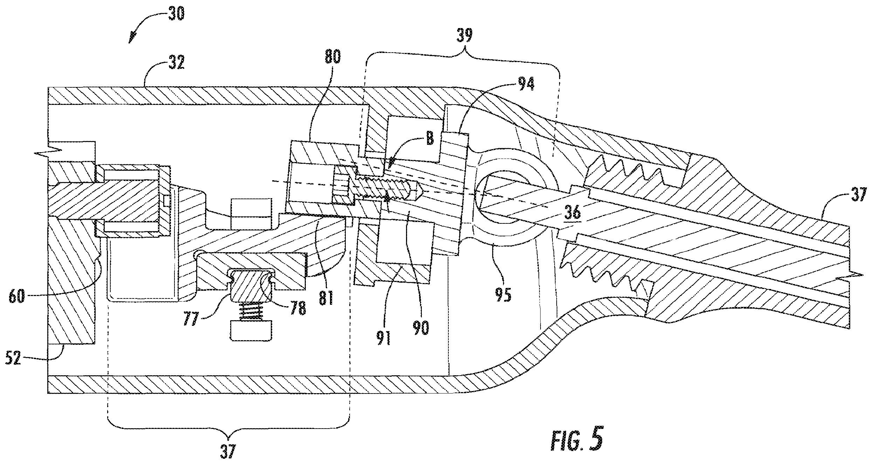

The outer race 66 is coupled to an oscillating connector 73, as for example with a pair of opposed pivot pins 74 projecting outwardly from the outer race and each being received in a respective bore 75 in a respective boss 76. The connector 73 is restrained in movement to a plane. In one example, a guide 77 (Fig. 5) is secured to the housing 32. The guide 77 is curved, and is received in a similarly curved slot 78 cooperating with the driver 37. Thus, the outer race 66 can only move in an oscillating manner, as can the connector 73. Another means to mount the connector 73 is with a pivot pin secured to the housing 32 and extending through a web portion 79 of the connector 73, which allows the connector 73 to rotate in an oscillating manner. The illustrated connector 73 has a curved gear rack portion 81, preferably a sector gear, coupled to the web 79 and carried thereby. A gear or gear segment, herein a gear 80, such as a bevel gear, engages the rack portion 81 of the driver 73 and itself is driven in an oscillating manner by rotation of the inner race 65 as driven by the motor 52. The gear 80 is coupled to the shaft 36 by the second driver 39 to effect driving of the shaft 36 in an oscillating manner.

The angle A determines the degree of rotation of the gear 80, and the rotational speed of the motor 52 determines the oscillation rate of the gear 80.

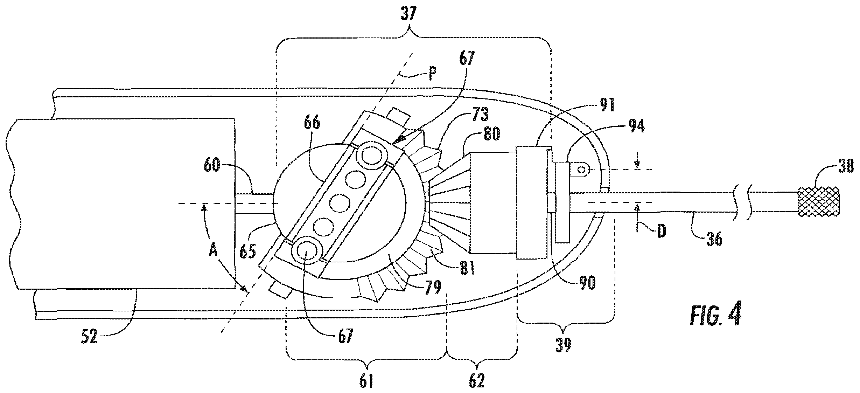

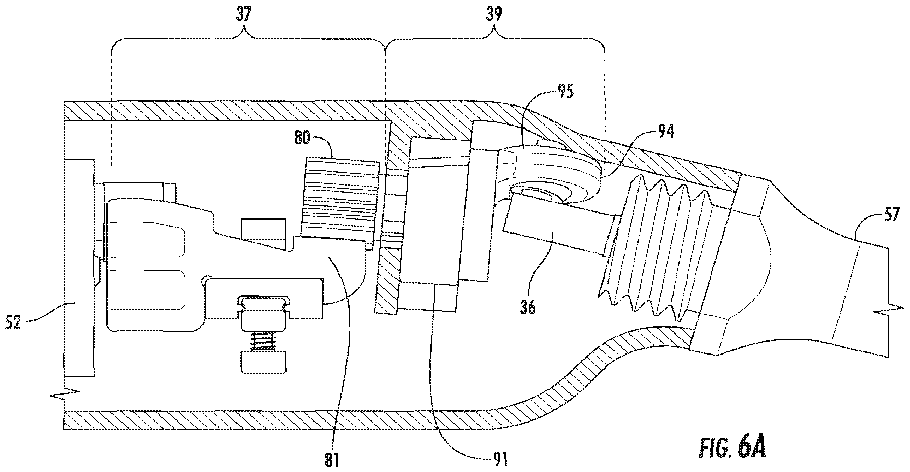

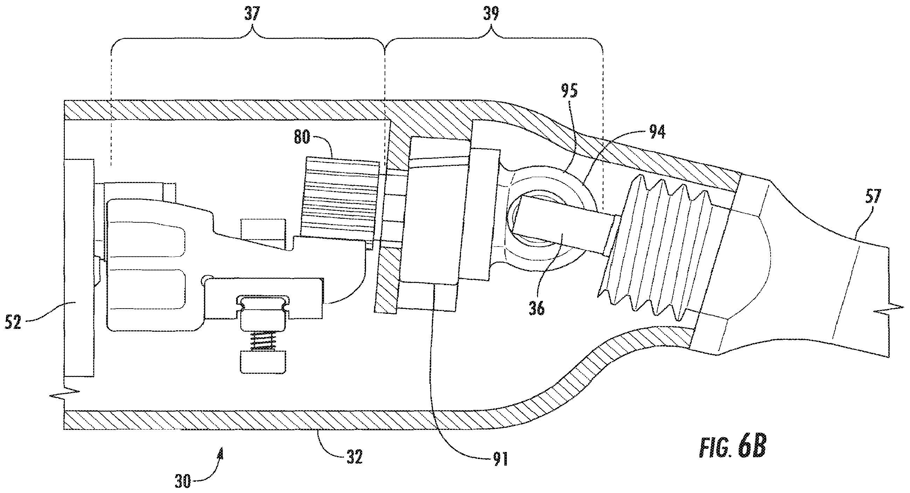

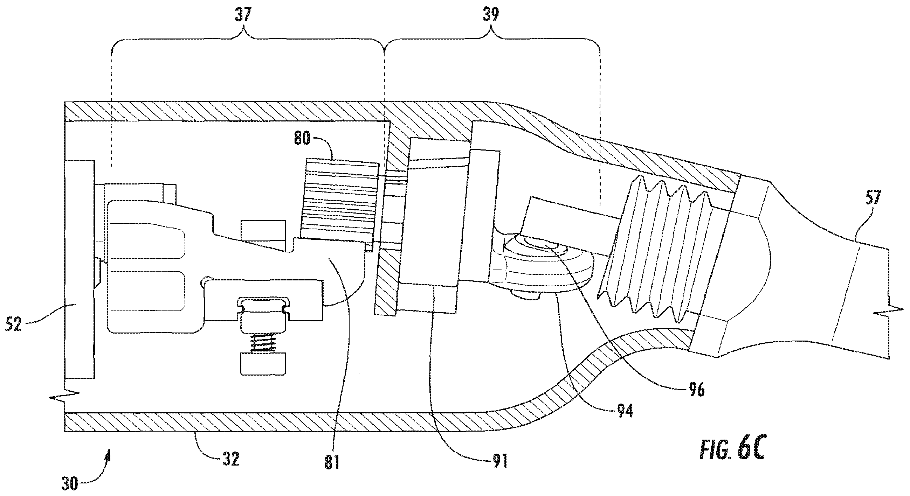

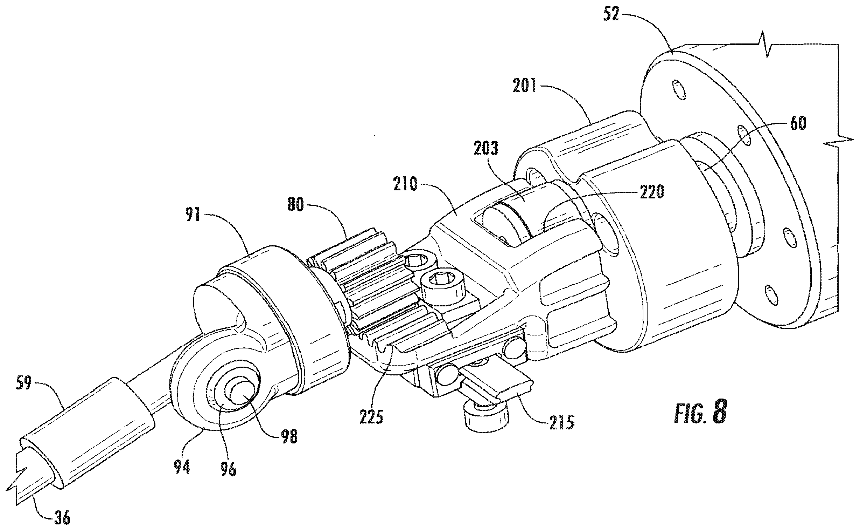

The gear 80 is part of the second driver 39, and is coupled to the shaft 36 to effect motion of the shaft 36 and associated cutting tool 38 as described herein. As shown, the gear 80 is fixed to a shaft 90 that is rotatably mounted to the housing 32 via a suitable bearing 91 fixed in position in the housing 32. The gear 80 is maintained in driving engagement with the rack 81, which oscillates along a curved path during operation of the motor 52. The shaft 36 is secured to a reciprocation effecting joint 94 in a manner allowing part of the joint 94 to pivot during rotation of the joint 94 and shaft 36. See Figs. 6A-6C. Oscillation of the shaft 90 and the joint 94 effects oscillation of the shaft 36. The longitudinal axis of the shaft 90 intersects the longitudinal axis of the shaft 36, Figs. 4, 5, and the axes are positioned at an angle B relative to one another. By being positioned at an angle B, which is preferably in the range of between about 5° and about 45°, the shaft 36, during oscillating rotation, will move longitudinally in two directions, effecting reciprocal movement of the shaft 36 and cutting tool 38 during their oscillating movement. To allow for both oscillation and reciprocation, the shaft 36 can be mounted in one or more journal bearings 59 fixed in position in the housing 32 and/or nose 57. The joint 94 acts as a wobble plate because of the angle B. Additionally, to effect the reciprocating movement, the shaft 36 is secured to the joint 94 at a position offset radially outwardly from the center of its rotation, the center of the shaft 90, Figs. 4, 6A. This offset dimension D also determines the amount of reciprocating movement of the shaft 36. In a preferred embodiment, the joint 94 oscillates about 180° and starts at a rotational position, where the shaft 36 is at its most retractable position and ends at its most extendable position. The joint 94, as shown, includes a tab 95 on which is mounted a ball or spherical bearing 96. The shaft 36 is coupled to the bearing 96 as with a pin 98, Fig. 8. Figs. 6A-6C illustrate the joint 94 in three different rotary positions and three different reciprocating positions. In Fig. 6A, the shaft is in its most extended reciprocating position. Fig. 6B shows the shaft 36 in an intermediate extended position. Fig. 6C shows the shaft 36 in its most retracted reciprocating position.

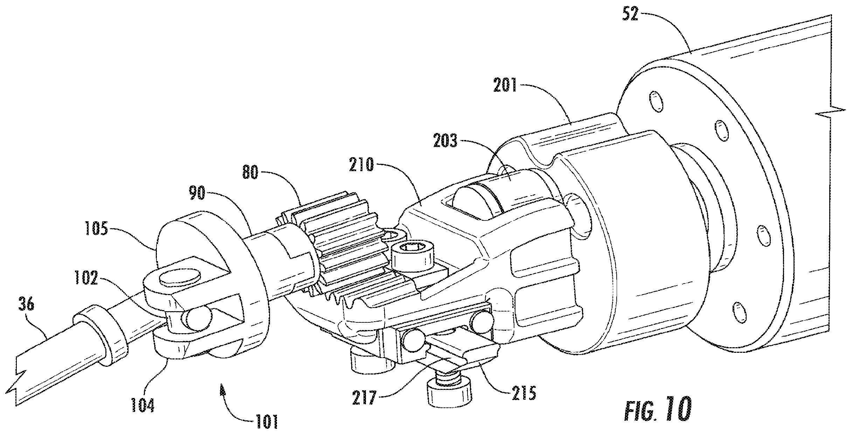

Fig. 10 illustrates another embodiment of connecting the shaft 36 to the second driver 39. The reciprocating effecting joint 101 is used instead of the joint 94. A pivot pin 102 is mounted for rotation in a clevis 104, which in turn is mounted to a crank member 105. The crank member 105 is mounted to a shaft 90, which is rotatably mounted in the bearing 91 as described above. The shaft 36 is secured to the pivot pin 102. This form of joint 101 is similar in operation to the joint 94 as described above.

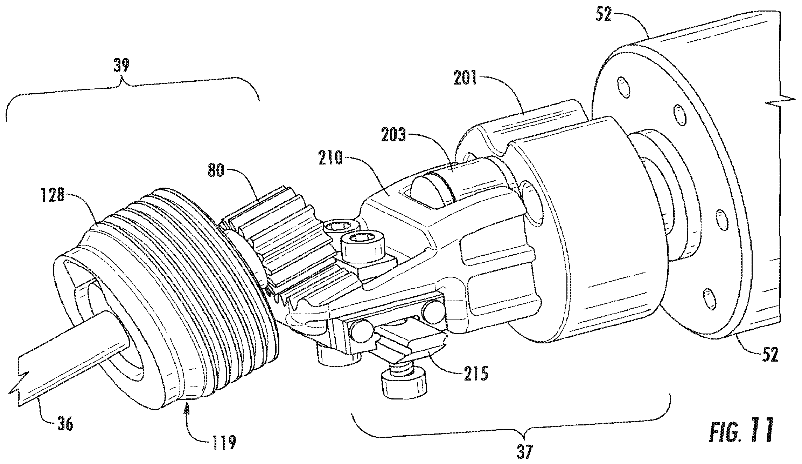

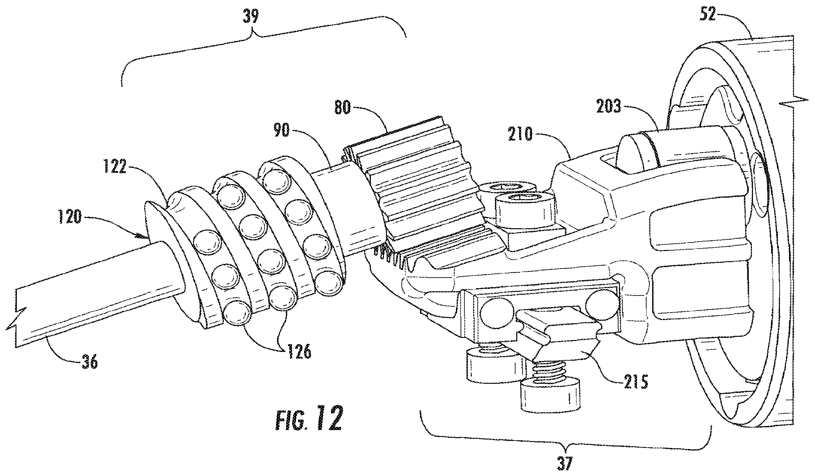

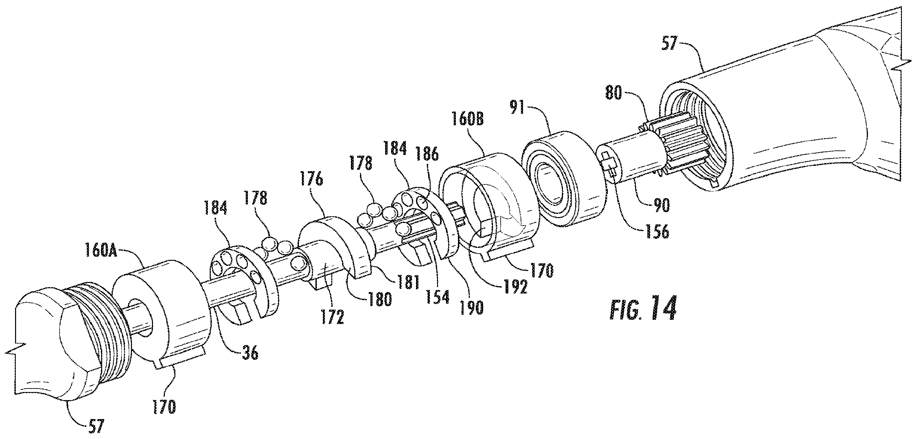

Figs. 11, 12 illustrate another embodiment of a second driver 39 that is operable to effect longitudinal reciprocating movement of the shaft 36. The shaft 36 is coupled to the shaft 90 relative to longitudinal movement therebetween, as for example, by the use of a spline connection, as can be seen in Fig. 14. An inner bearing race 120 is secured to the shaft 36 and has an outwardly opening helical bearing groove 122. A plurality of bearing balls 126 are contained within the groove 122. An outer bearing race 128 is mounted in the housing 32 or nose 57 and is fixed against movement relative thereto. The outer bearing race 128 has a helical groove (not shown) that opens inwardly and contains the bearing balls 126 therein. When the shaft 36 rotates in an oscillating manner, as effected by the first driver 37, the shaft 36 will move in a longitudinal reciprocating manner by cooperation between the inner and outer bearing races 120, 128, respectively, via the bearing balls 126. This forces the inner race 120 to move longitudinally in a reciprocating manner.

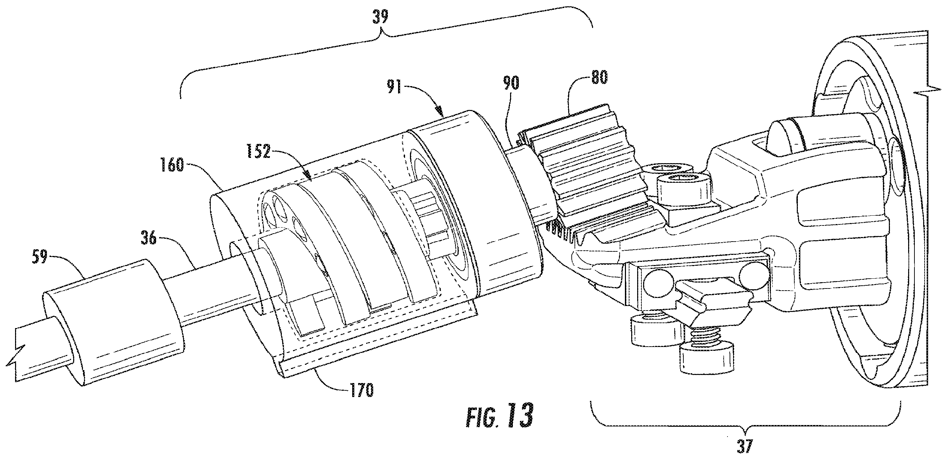

Figs. 13-16 illustrate a further embodiment of a second driver 39 that is operable to effect longitudinal reciprocating movement of the shaft 36. This embodiment uses a helical bearing 152 to effect longitudinal reciprocating movement of the shaft 36 while the shaft 36 is being rotationally oscillated by the first driver 37. As seen in Fig. 14, the shaft 36 has its proximal end 154 male splined and is longitudinally movably received in a female splined socket 156 within the shaft 90. Thus, the shaft 36 can move both longitudinally and rotationally while being driven by the drivers 37, 39. The helical bearing 152 includes a split housing 160 having housing portions 160A and 160B. The housing 160 is mounted in the housing 32 and/or its nose 57 in a manner to prevent relative rotation therebetween. This can be accomplished, as seen in Figs. 13, 14 by providing the housing 160 with a laterally projecting key 170. The bearing 152 has an inner race 172 secured to the shaft 36 and provides a radially projecting helically longitudinally extending flange 176. Bearing balls 178 are positioned on opposite faces 180, 181 of the flange 176. The helical bearing 152 is provided with a pair of outer races 184 that have a plurality of bearing ball receiving pockets 186 in the faces opposite the faces 180, 181. The outer races 184 retain the bearing balls 178 in contact with their respective face 180 or 181. Rotation of the outer races 184 relative to the housing portions 160A and 160B is limited by stop faces 190 on the outer races 184, and stop faces 192 on the inside of the housing portions 160A and 160B.

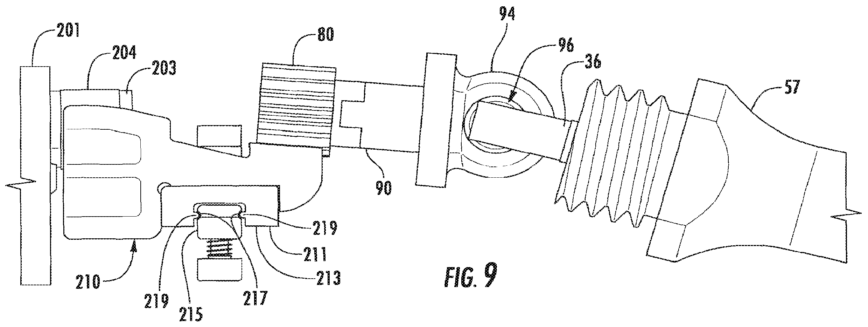

Figs. 8, 9 illustrate a second embodiment of the first driver 37. It is similar to the driver 37 shown in Figs. 5, 6A-6C. The motor 52 has a crank assembly 201 mounted on its output shaft 60. The crank assembly 201 includes a drive arm 203 that can include a wear resistant bearing member 204. The drive arm 203 is offset radially from the center of rotation of the crank assembly 201. Thus, rotation of the crank assembly 201 moves the drive arm 203 in a circular path. A follower assembly 210 is mounted in the housing 32 in a manner to restrict its movement in a plane laterally from side to side. As shown, a guide bed 211 is provided and includes a guide channel 213, which receives in it a guide rail 215. As shown, the guide rail 215 is coupled to the bed 211 to prevent their separation during movement. As illustrated, the guide rail 215 has a pair of opposed grooves 217 in each of which is received a respective guide rail 219 to provide guided restrained movement between the guide bed 211 and guide rail 215. The guide rail 215 is straight, thereby restricting movement of the follower assembly to linear movement in a plane. The drive arm 203 is received in a channel 220 with a close fit, whereupon revolving movement of the drive arm 203 will effect reciprocating lateral movement of the follower assembly 210. The follower assembly 120 is drivingly coupled to the second driver 39 in a manner to effect oscillating rotation of the shaft 36. As shown, a gear rack 225 is provided on the follower assembly 210 to mesh with the gear 80, whereby lateral movement of the follower assembly 210 effects oscillating rotation of the shaft 36, which, with operation of the second driver 39, will simultaneously effect reciprocating motion of the shaft 36.

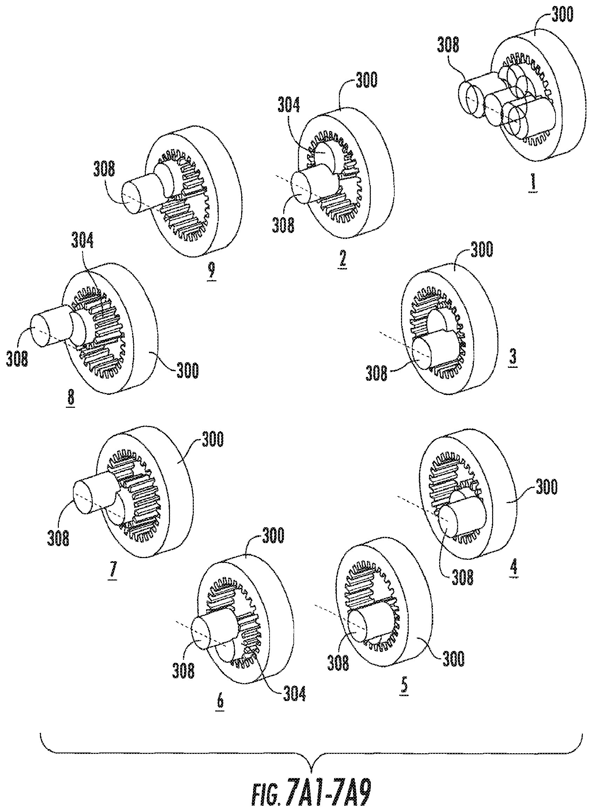

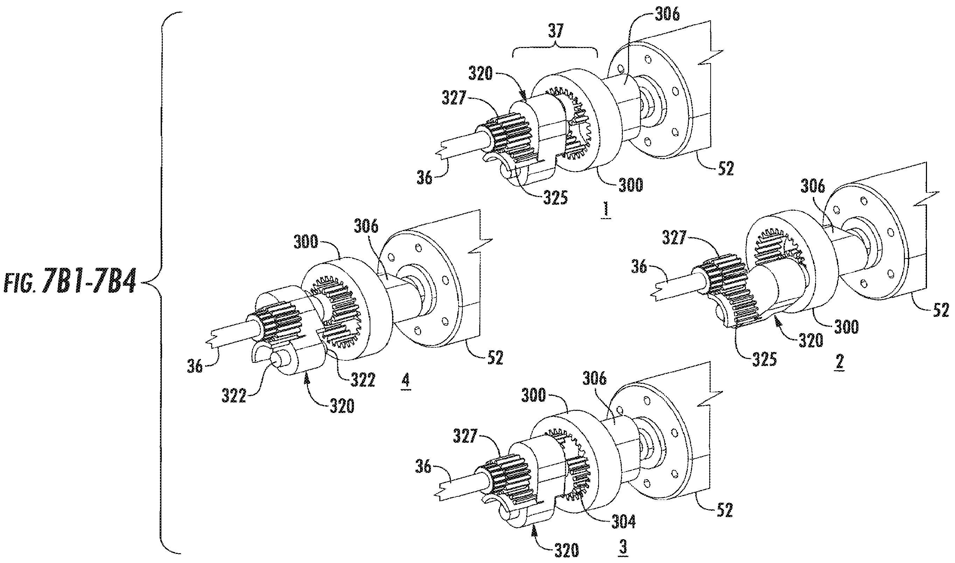

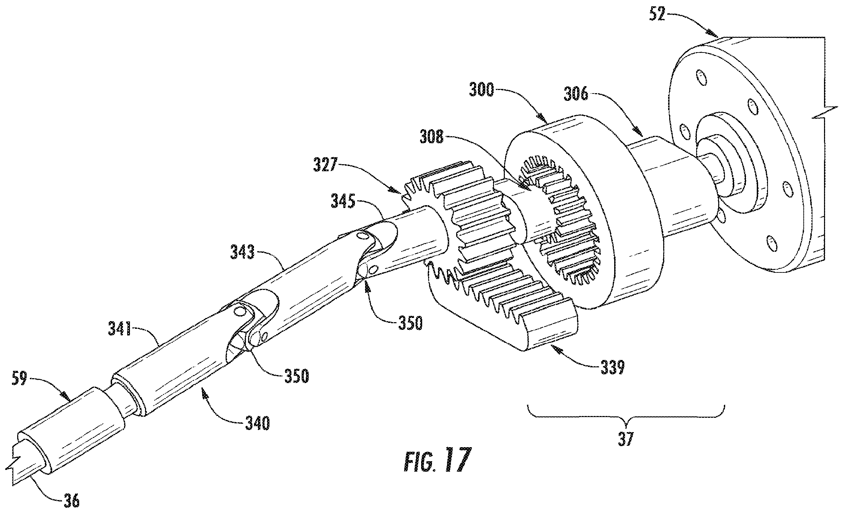

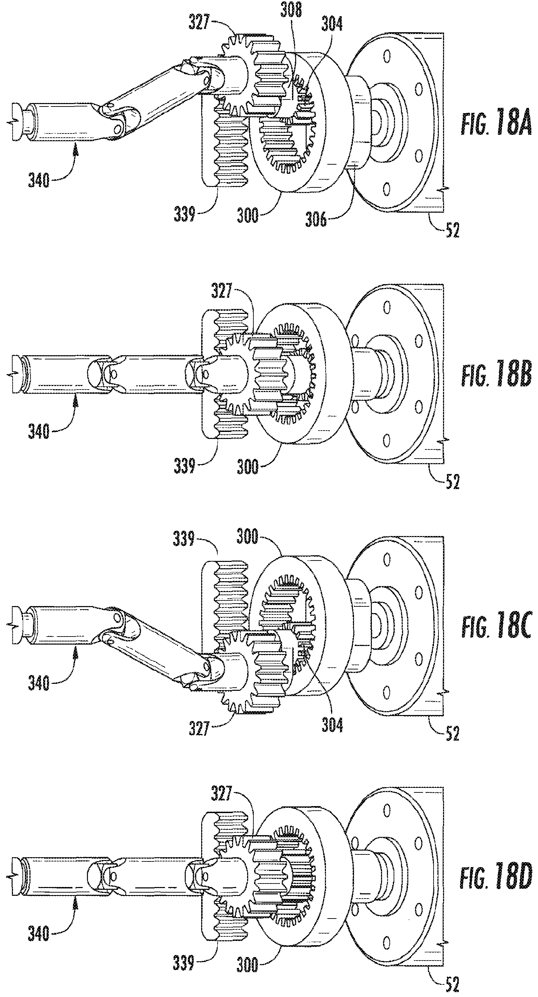

Figs. 7A, 7B illustrate another form of drivers 37, 39. The first driver 37 is illustrated as a Cardan type drive that is operable to effect rotary oscillation of the shaft 36. While the structure shown in these Figures effects only oscillating rotation, the additional structure shown in Figs. 17, 18A-18D shows a mechanism to convert the oscillating rotation into oscillating rotation and linear reciprocation of the shaft 36.

Fig. 7A illustrates the basic functioning of a Cardan mechanism. An internal gear member 300 has an external gear 304 received therein. The gear ratio between the internal gear 300 and the external gear 304 is 2:1. The gear 300, in this case, is fixed against movement, while the gear 304 is part of a crank arm 306 mounted to motor 52. As the crank arm 306 effects revolving of the gear 304 about the center of rotation of the motor shaft, the gear 304 moves about the interior of the internal gear 300. The gear 304 has secured thereto an output arm 308 that has a center of rotation that is coaxial with the center of rotation of the motor 52 when the arm 308 is at its center position within the gear 300, as seen in Fig. 7A2-7A9. In this type of mechanism, the center of the arm 308 moves in a linear path in a laterally reciprocating manner. Thus, rotary output motion of the motor shaft can be converted into reciprocating linear motion. This can be seen in Fig. 17.

As seen in Fig. 7B, the Cardan style first driver 37 is coupled to a follower 320 that is operable to convert the linear movement of the arm 308 into oscillating rotary motion of the shaft 36. The illustrated follower 320 receives the arm 308 in an elongate slot (not shown) on the side facing the motor 52; this allows the arm 308 to move freely as the follower 320 pivots about a pair of pivot pins 322 that are mounted in suitable bearings (not shown) in the housing 32 and/or its nose 57. As the arm 308 moves laterally, as seen in Fig. 7A, it will force the follower 320 to pivot. A curved gear rack 325 is secured to the follower 320, is preferably integral therewith, and has the gear teeth spaced radially outwardly from the pivot pins 322. The radius of the gear rack 325 is substantially the radial distance of the gears from the center of rotation of the pivot pins 322. The gear rack 325 is meshed with a gear or gear segment 327, such as a spur gear that is secured to the shaft 36. As the follower 320 oscillates about its pivot pins 322, the shaft 36 is driven in a rotary oscillating manner.

Figs. 17 and 18A-18D illustrate a still further embodiment of a second driver 39. It utilizes a Cardan first driver 37, such as shown in Fig. 7B. However, instead of a curved gear rack 325, this form uses a straight gear rack 339, and the gear 327 which is coupled to the shaft 36 moves laterally with its center of rotation being in a straight line. This can be accomplished by having the arm 308 centered on the center of rotation of the gear 304. The gear 327 is coupled to the shaft 36 through the use of a drive shaft 340. As shown, the drive shaft 340 has three sections 341, 343, and 345. Section 341 is secured to the shaft 36, which, in turn, is mounted in the bearing 59, as described above. Section 343 is coupled to section 341 in a manner that allows the axes of sections 341 and 343 to change their angular orientation. This can be accomplished by a universal joint (u-joint) 350. Section 343 is coupled to section 345 in a similar manner, as with a second universal joint 350. As the gear 327 rotates and moves laterally side-by-side on the gear rack 339, the length of the drive shaft 340 increases and decreases, effecting linear reciprocating movement of the shaft 36. This can be seen in Figs. 18 A-D.

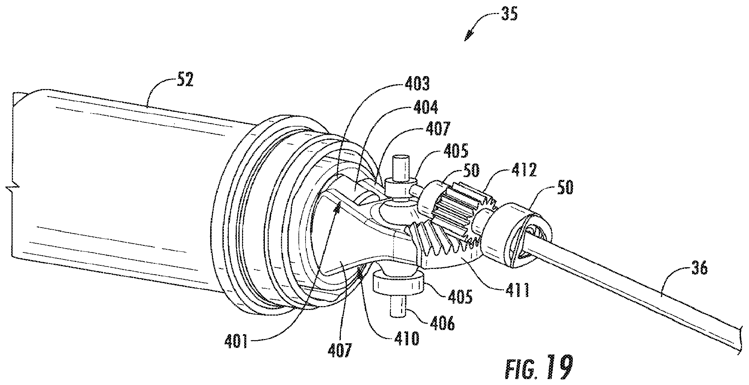

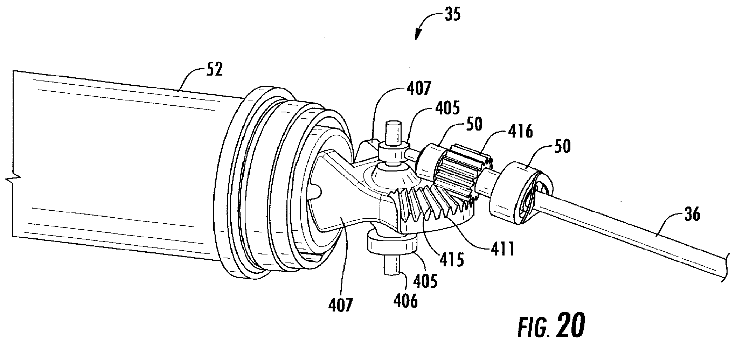

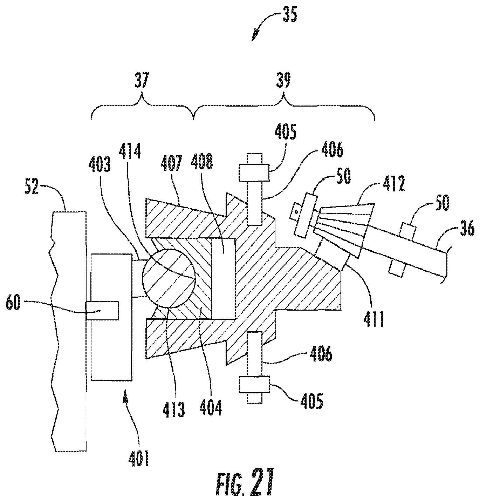

Figs. 19-21 illustrate another embodiment of the transmission 35, first driver 37 and second driver 39. The transmission 35 in Figs. 19-21 is similar to that shown in Figs. 5, 6A-6C in that it uses both a rack and pinion gear drive arrangement and a crank assembly. The motor 52, described above, has a crank assembly 401 mounted on its output shaft 60. The crank assembly 401 includes a drive arm 403 that can include a wear resistant bearing member 404. The drive arm 403 is offset radially from the center of rotation of the crank assembly 401. Thus, rotation of the crank assembly 401 moves the drive arm 403 in a circular path. A follower assembly 410 is mounted in the housing 32 in a manner to restrict its movement in a plane laterally from side to side in a pivoting manner about an axle arrangement 406. The axle arrangement 406 is mounted for pivoting movement of follower assembly 410 with suitable bearings 405 mounted in the housing 32. The follower assembly 410 has a pair of spaced apart arms 407, each with an inwardly opening channel 408 sized and shaped to receive the bearing member 404 therein. The channels 408 are portions of a cylinder and the bearing 404 is a cylinder, allowing the bearing 404 to move both longitudinally and rotationally relative to the follower assembly 410. The bearing 404 is mounted to the drive arm 403 in a manner to allow the drive arm to be rotated by the motor 52 and effect rotational pivoting movement of the follower assembly 410. As shown, the drive arm 403 is provided with a generally spherical bearing 413 mounted in a spherical cavity 414 in the bearing 404 that permits multi axis rotation of the bearing 413 relative to the bearing 404. The bearing 404 is in the form of a ball joint. When the drive arm 403 is driven so the bearing 413 moves in a circular path, the bearing moves longitudinally in the channels 408, as well as rotationally. The follower assembly 410 is provided with a gear rack 411 forward of the axle arrangement 406 from the arms 407. The rack 411 is preferably a sector gear and is preferably curved, having an inner edge curved in a circular arc with a radius approximately equal to its spacing from the center of rotation about the axle assembly 406 and an outer edge curved in an arc with a radius approximately equal to its spacing from the center of rotation about the axle assembly 406. The gear tooth surface 415 is beveled relative to the plane of rotation of the follower assembly 410. This accommodates its driving a pinion gear 416 mounted to the shaft 36 to which it is mounted. The gear 416 is a bevel gear that has gear teeth that mesh with the gear teeth of the rack 411. The shaft 36 is mounted in the housing 32 via bearings 50 as described above. The rack 411 rotates in two directions about the axle assemble 406 which effects oscillating rotation of the shaft 36, also in two directions. Thus, the follower assembly 410 converts one directional rotation of the motor 52 and drive arm 403 into two direction oscillatory rotation.

The term gear, bevel gear, curved gear rack and gear rack as used herein includes both complete gears and gear segments.

It is to be understood that while a certain form of the invention is illustrated, it is not to be limited to the specific form or arrangement herein described and shown. It will be apparent to those skilled in the art that various changes may be made without departing from the scope of the invention, and the invention is not to be considered limited to what is shown and described in the specification and any drawings/figures included herein.

One skilled in the art will readily appreciate that the present invention is well adapted to carry out the objectives and obtain the ends and advantages mentioned, as well as those inherent therein. The embodiments, procedures and techniques described herein are presently representative of the preferred embodiments, are intended to be exemplary, and are not intended as limitations on the scope. Changes therein and other uses will occur to those skilled in the art which are encompassed within the scope of the invention and are defined by the scope of the appended claims. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A surgical tool (30) having a cutter (38) that rotary oscillates and linearly reciprocates, said surgical tool (30) including:
- a housing (32) forming a handle (34);
- a motor (52) mounted in the housing (32) and having a rotatable output shaft (60);
- a transmission mounted in the housing (32) and coupled to the motor (52) output shaft (60) for selectively driving the transmission (35);
- a cutter shaft (36) carried by the housing (32) and having a cutter (38) on a distal end thereof, said shaft (36) being mounted for simultaneous oscillating rotation and linear reciprocation, said cutter shaft (36) being coupled to the transmission (35) for effecting oscillating and reciprocating movement of the cutter shaft (36); and wherein
- the transmission (35) having a first driver (37) coupled to the motor output shaft (60) and operable to effect rotary oscillation of the cutter shaft (36), said transmission (35) having a second driver (39) coupled to the first driver (37) operationally downstream thereof, said second driver (39) being coupled to the cutter shaft (36) and being operable to effect linear reciprocation of the cutter shaft (36) and transmit the rotary oscillation driving from the first driver (37) to the cutter shaft (36), **characterized in that** the first driver (37) includes:
- a crank (401) mounted on the output shaft (60) of the motor and including a drive arm (403) that includes a wear resistant bearing member (404) and that is offset radially from the center of rotation of the crank assembly (401) so that rotation of the crank assembly (401) moves the drive arm (403) in a circular path;
- the drive arm (403) being provided with a generally spherical bearing (413) mounted in a spherical cavity (414) in the bearing member (404) that permits multi axis rotation of the bearing (413) relative to the bearing (404);
- a follower assembly (410) mounted in the housing (32) in a manner to restrict its movement in a plane laterally from side to side in a pivoting manner about an axle arrangement (406) wherein the axle arrangement (406) is mounted for pivoting movement of follower assembly (410) with suitable bearings (405) mounted in the housing (32)
- wherein the follower (410) assembly has a pair of spaced apart arms (407) each with an inwardly opening channel (408) sized and shaped to receive the bearing member (404) therein,
- wherein the channels (408) are portions of a cylinder and the bearing (404) is a cylinder, allowing the bearing (404) to move both longitudinally and rotationally relative to the follower assembly (410), the bearing (404) being mounted to the drive arm (403) in a manner to allow the drive arm (403) to be rotated by the motor (52) and effect rotational pivoting movement of the follower assembly (410), the bearing (104) being in the form of a ball joint that when the drive arm (403) is driven so the bearing (413) moves in a circular path, the bearing (404) moves longitudinally in the channels (408), as well as rotationally,
- wherein the follower assembly (410) is further provided with a gear rack (411) forward of the axle arrangement (406) from the arms (407) having a gear tooth surface (415) that is beveled relative to the plane of rotation of the follower assembly (410), the follower assembly (410) accommodates its driving a pinion gear (416) mounted to the shaft (36) to which it is mounted, the gear (416) being a bevel gear that has gear teeth that mesh with the gear teeth of the rack (411), the shaft (36) being mounted in the housing (32) via bearings (50) and wherein the rack (411) rotates in two directions about the axle assembly (406) which effects oscillating rotation of the shaft (36), also in two directions so that the follower assembly (410) converts one directional rotation of the motor (52) and drive arm (403) into two direction oscillatory rotation.

2. The surgical tool (30) of claim 1 wherein said first driver (37) being positioned between said motor (52) and said second driver (39) and being operable to drive said second driver (39).

3. The surgical tool (30) of claim 2 wherein the follower of the first driver (37) includes a curved gear rack pivotally mounted in the housing (32) for oscillating rotational movement and said second driver (39) includes a bevel gear meshed with said curved gear rack, said first driver (37) also including a first bearing race member with a first bearing race and a second bearing race member with a second bearing race, said second bearing race member being coupled to said gear rack and said first bearing race member and operable to effect pivoting movement of the gear rack upon rotation of said motor (52) output shaft (60), said first bearing race being positioned at an angle A in the range of between about 30° and about 80°, said pinion gear being coupled to said cutter (38) shaft.

4. The surgical tool (30) of claim 3 wherein said second driver (39) including a reciprocation effecting joint coupling said cutter (38) shaft to said second driver (39), said bevel gear being mounted on a first shaft for rotation therewith about a first axis of rotation and said cutter (38) shaft having a second axis of rotation with the second axis of rotation and said first axis of rotation being at an angle B of between about 5° and about 45° to effect reciprocation of the cutter (38) shaft during rotation of said motor (52) output shaft (60).

5. The surgical tool of any one of the preceding claims wherein the rack (411) is a sector gear.

6. The surgical tool of the preceding claim wherein the rack (411) is curved, having an inner edge curved in a circular arc with a radius approximately equal to its spacing from the center of rotation about the axle assembly (406) and an outer edge curved in an arc with a radius approximately equal to its spacing from the center of rotation about the axle assembly (406).

## Patentansprüche

1. Chirurgisches Instrument (30) mit einem Schneidwerkzeug (38), das rotierend oszilliert und sich linear hin- und herbewegt, wobei das chirurgische Instrument (30) umfasst:
- ein Gehäuse (32), das einen Griff (34) bildet;
- einen Motor (52), der im Gehäuse (32) befestigt ist und eine drehbare Ausgangswelle (60) aufweist;
- ein Getriebe, das im Gehäuse (32) befestigt und mit der Ausgangswelle (60) des Motors (52) verbunden ist, um das Getriebe (35) selektiv anzutreiben;
- eine durch das Gehäuse (32) abgestützte Schneidwelle (36) mit einem Schneidwerkzeug (38) an ihrem distalen Ende, wobei die Welle (36) für eine gleichzeitige oszillierende Drehung und lineare Hin- und Herbewegung befestigt ist, wobei die Schneidwelle (36) mit dem Getriebe (35) verbunden ist, um eine oszillierende und eine hin- und hergehende Bewegung der Schneidwelle (36) zu bewirken; und wobei
- das Getriebe (35) einen ersten Antrieb (37) aufweist, der mit der Motorausgangswelle (60) verbunden ist und betätigbar ist, um eine Drehschwingung der Schneidwelle (36) zu bewirken, wobei das Getriebe (35) einen zweiten Antrieb (39) aufweist, der mit dem ersten Antrieb (37) funktionsmäßig stromabwärts davon verbunden ist, wobei der zweite Antrieb (39) mit der Schneidwelle (36) verbunden ist und betätigbar ist, um eine lineare Hin- und Herbewegung der Schneidwelle (36) zu bewirken und die Drehschwingung vom ersten Antrieb (37) auf die Schneidwelle (36) zu übertragen, **dadurch gekennzeichnet, dass** der erste Antrieb (37) umfasst:
- eine Kurbel (401), die auf der Ausgangswelle (60) des Motors befestigt ist und einen Antriebsarm (403) umfasst, der ein verschleißfestes Lagerelement (404) umfasst und radial vom Drehzentrum der Kurbelanordnung (401) versetzt ist, so dass eine Drehung der Kurbelanordnung (401) den Antriebsarm (403) in einer kreisförmigen Bahn bewegt;
- wobei der Antriebsarm (403) mit einem im Wesentlichen kugelförmigen Lager (413) versehen ist, das in einer kugelförmigen Ausnehmung (414) im Lagerelement (404) befestigt ist, das eine mehrachsige Drehung des Lagers (413) relativ zum Lager (404) ermöglicht;
- eine Mitnehmeranordnung (410), die im Gehäuse (32) derart befestigt ist, um ihre Bewegung in einer Ebene seitlich von einer Seite zur anderen schwenkbar um eine Achsenanordnung (406) herum einzuschränken, wobei die Achsenanordnung (406) für eine Schwenkbewegung der Mitnehmeranordnung (410) mit geeigneten Lagern (405) befestigt ist, die im Gehäuse gelagert sind (32)
- wobei die Mitnehmeranordnung (410) ein Paar voneinander beabstandeter Arme (407) aufweist, wobei jeder einen sich nach innen öffnenden Kanal (408) aufweist, der dimensioniert und ausgebildet ist, um das Lagerelement darin aufzunehmen,
- wobei die Kanäle (408) Teile eines Zylinders sind und das Lager (404) ein Zylinder ist, wodurch sich das Lager (404) sowohl in Längsrichtung als auch in Drehrichtung relativ zur Mitnehmeranordnung (410) bewegen kann, wobei das Lager (404) derart am Antriebsarm (403) befestigt ist, dass der Antriebsarm (403) durch den Motor (52) gedreht werden kann und eine Drehschwenkbewegung der Mitnehmeranordnung (410) bewirken kann, wobei das Lager (104) die Form eines Kugelgelenks aufweist, dass das Lager (404) sowohl in Längsrichtung in den Kanälen (408) als auch rotatorisch bewegt, wenn der Antriebsarm (403) derart angetrieben wird, dass sich das Lager (413) auf einer Kreisbahn bewegt,
- wobei die Mitnehmeranordnung (410) ferner mit einer Zahnstange (411) vor der Achsenanordnung (406) von den Armen (407) versehen ist, die eine Zahnradfläche (415) aufweist, die relativ zur Drehebene der Mitnehmeranordnung (410) abgeschrägt ist, wobei die Mitnehmeranordnung (410) ihren Antrieb über ein an der Welle (36) befestigtes Ritzel (416) aufnimmt, an der sie befestigt ist, wobei das Zahnrad (416) ein Kegelrad ist, das Zahnradzähne aufweist, die mit den Zahnradzähnen der Zahnstange (411) in Eingriff stehen, wobei die Welle (36) über Lager (50) im Gehäuse (32) befestigt ist und wobei sich die Zahnstange (411) in zwei Richtungen um die Achsenanordnung (406) dreht, was eine oszillierende Drehung der Welle (36) auch in zwei Richtungen bewirkt, so dass die Mitnehmeranordnung (410) eine unidirektionale Drehung des Motors (52) und des Antriebsarms (403) in eine oszillierende Drehung in zwei Richtungen umwandelt.

2. Chirurgisches Instrument (30) nach Anspruch 1, wobei der erste Antrieb (37) zwischen dem Motor (52) und dem zweiten Antrieb (39) positioniert ist und zum Antreiben des zweiten Antriebs (39) betätigbar ist.

3. Chirurgisches Instrument (30) nach Anspruch 2, wobei der Mitnehmer des ersten Antriebs (37) eine gebogene Zahnstange umfasst, die schwenkbar im Gehäuse (32) für eine oszillierende Drehbewegung befestigt ist, und der zweite Antrieb (39) ein Kegelrad umfasst, das mit der gebogenen Zahnstange in Eingriff steht, wobei der erste Antrieb (37) auch ein erstes Lagerringelement mit einem ersten Lagerring und ein zweites Lagerringelement mit einem zweiten Lagerring umfasst, wobei das zweite Lagerringelement mit der Zahnstange und dem ersten Lagerringelement verbunden ist und betätigbar ist, um eine Schwenkbewegung der Zahnstange bei Drehung der Ausgangswelle (60) des Motors (52) zu bewirken, wobei der erste Lagerring in einem Winkel A im Bereich zwischen etwa 30° und etwa 80° positioniert ist und das Ritzel mit dem Schneidwerkzeug (38) verbunden ist.

4. Chirurgisches Instrument (30) nach Anspruch 3, wobei der zweite Antrieb (39) ein eine Hin- und Herbewegung bewirkendes Gelenk umfasst, das das Schneidwerkzeug (38) mit dem zweiten Antrieb (39) verbindet, wobei das Kegelrad auf einer ersten Welle zur Drehung um eine erste Drehachse mit dieser gelagert ist und wobei das Schneidwerkzeug (38) eine zweite Drehachse aufweist, wobei die zweite Drehachse und die erste Drehachse in einem Winkel B zwischen etwa 5° und etwa 45° zueinander stehen, um eine Hin- und Herbewegung des Schneidwerkzeugs (38) während einer Drehung der Ausgangswelle (60) des Motors (52) zu bewirken.

5. Chirurgisches Instrument nach einem der vorhergehenden Ansprüche, wobei die Zahnstange (411) ein Zahnbogen ist.

6. Chirurgisches Instrument nach einem der vorhergehenden Ansprüche, wobei die Zahnstange (411) gebogen ist, eine Innenkante aufweist, die in einem Kreisbogen mit einem Radius gebogen ist, der ungefähr ihrem Abstand vom Drehzentrum um die Achsenanordnung (406) entspricht, und eine Außenkante aufweist, die in einem Bogen mit einem Radius gebogen ist, der ungefähr ihrem Abstand vom Drehzentrum um die Achsenanordnung (406) entspricht.

## Revendications

1. Outil chirurgical (30) comportant un organe de coupe (38) qui oscille de façon rotative et effectue un mouvement de va-et-vient linéaire, ledit outil chirurgical (30) comprenant :
- un boîtier (32) formant une poignée (34) ;
- un moteur (52) monté dans le boîtier (32) et comportant un arbre de sortie rotatif (60) ;
- une transmission montée dans le boîtier (32) et couplée à l'arbre de sortie (60) du moteur (52) pour entraîner sélectivement la transmission (35) ;
- un arbre de coupe (36) porté par le boîtier (32) et comportant un organe de coupe (38) à son extrémité distale, ledit arbre (36) étant monté pour une rotation oscillante et un mouvement de va-et-vient linéaire simultanés, ledit arbre de coupe (36) étant couplé à la transmission (35) pour effectuer un mouvement oscillant et de va-et-vient de l'arbre de coupe (36) ; et dans lequel la transmission (35) comporte un premier organe d'entraînement (37) couplé à l'arbre de sortie du moteur (60) et pouvant effectuer une oscillation rotative de l'arbre de coupe (36), ladite transmission (35) comportant un second organe d'entraînement (39) couplé au premier organe d'entraînement (37) en aval opérationnel de celui-ci, ledit second organe d'entraînement (39) étant couplé à l'arbre de coupe (36) et pouvant effectuer un mouvement de va-et-vient linéaire de l'arbre de coupe (36) et transmettre l'entraînement de l'oscillation rotative du premier organe d'entraînement (37) à l'arbre de coupe (36), **caractérisé en ce que** le premier organe d'entraînement (37) comprend :
- une manivelle (401) montée sur l'arbre de sortie (60) du moteur et comprenant un bras d'entraînement (403) qui comprend un palier (404) résistant à l'usure et qui est décalé radialement par rapport au centre de rotation de l'ensemble manivelle (401) de sorte que la rotation de l'ensemble manivelle (401) déplace le bras d'entraînement (403) dans une trajectoire circulaire ;
- le bras d'entraînement (403) étant muni d'un palier généralement sphérique (413) monté dans une cavité sphérique (414) du palier (404) qui permet une rotation multiaxiale du palier (413) par rapport au palier (404) ;
- un ensemble suiveur (410) monté dans le boîtier (32) de manière à limiter son mouvement dans un plan latéral d'un côté à l'autre d'une manière pivotante autour d'un arrangement d'axe (406) dans lequel l'arrangement d'axe (406) est monté pour le mouvement pivotant de l'ensemble suiveur (410) avec des paliers appropriés (405) montés dans le boîtier (32)
- dans lequel l'ensemble suiveur (410) comporte une paire de bras (407) espacés, chacun doté d'un canal (408) s'ouvrant vers l'intérieur, dimensionné et formé pour recevoir le palier (404) à l'intérieur,
- dans lequel les canaux (408) sont des parties d'un cylindre et le palier (404) est un cylindre, permettant au palier (404) de se déplacer à la fois longitudinalement et en rotation par rapport à l'ensemble suiveur (410), le palier (404) étant monté sur le bras d'entraînement (403) de manière à permettre au bras d'entraînement (403) d'être entraîné en rotation par le moteur (52) et d'effectuer un mouvement de rotation et de pivotement de l'ensemble suiveur (410), le palier (104) se présentant sous la forme d'un joint à rotule qui, lorsque le bras d'entraînement (403) est entraîné de manière à ce que le palier (413) se déplace sur une trajectoire circulaire, le palier (404) se déplace longitudinalement dans les canaux (408), ainsi que dans le sens de la rotation,
- l'ensemble suiveur (410) est en outre pourvu d'une crémaillère (411) située à l'avant de l'arrangement d'axe (406) des bras (407) ayant une surface de dent d'engrenage (415) qui est biseautée par rapport au plan de rotation de l'ensemble suiveur (410), l'ensemble suiveur (410) entraîne un pignon (416) monté sur l'arbre (36) sur lequel il est monté, l'engrenage (416) étant un engrenage conique dont les dents d'engrenage s'engrènent avec celles de la crémaillère (411), l'arbre (36) étant monté dans le boîtier (32) par l'intermédiaire de paliers (50) et dans lequel la crémaillère (411) tourne dans deux directions autour de l'ensemble d'axe (406), ce qui entraîne une rotation oscillante de l'arbre (36), également dans deux directions, de sorte que l'ensemble suiveur (410) convertit la rotation unidirectionnelle du moteur (52) et du bras d'entraînement (403) en une rotation oscillatoire bidirectionnelle.

2. Outil chirurgical (30) selon la revendication 1 dans lequel ledit premier organe d'entraînement (37) est positionné entre ledit moteur (52) et ledit second organe d'entraînement (39) et peut entraîner ledit second organe d'entraînement (39).

3. Outil chirurgical (30) selon la revendication 2 dans lequel le suiveur du premier organe d'entraînement (37) comprend une crémaillère incurvée montée pivotante dans le boîtier (32) pour un mouvement rotatif oscillant et ledit second organe d'entraînement (39) comprend un engrenage conique engrené avec ladite crémaillère incurvée, ledit premier organe d'entraînement (37) comprenant également un premier élément de chemin de roulement avec un premier chemin de roulement et un second élément de chemin de roulement avec un second chemin de roulement, ledit second élément de chemin de roulement étant couplé à ladite crémaillère et audit premier élément de chemin de roulement et pouvant effectuer un mouvement de pivotement de la crémaillère lors de la rotation de l'arbre de sortie (60) dudit moteur (52), ledit premier chemin de roulement étant positionné à un angle A compris entre environ 30° et environ 80° et ledit pignon étant couplé audit arbre de l'organe de coupe (38).

4. Outil chirurgical (30) selon la revendication 3, dans lequel ledit second organe d'entraînement (39) comprend un joint à effet de mouvement de va-et-vient qui relie ledit arbre de l'organe de coupe (38) audit second organe d'entraînement (39), ledit engrenage conique étant monté sur un premier arbre pour tourner autour d'un premier axe de rotation et ledit arbre de l'organe de coupe (38) ayant un second axe de rotation, le second axe de rotation et ledit premier axe de rotation étant à un angle B compris entre environ 5° et environ 45° pour effectuer un mouvement de va-et-vient de l'arbre de l'organe de coupe (38) pendant la rotation dudit arbre de sortie (60) du moteur (52).

5. Outil chirurgical selon l'une quelconque des revendications précédentes dans lequel la crémaillère (411) est un secteur denté.

6. Outil chirurgical selon la revendication précédente dans lequel la crémaillère (411) est incurvée, ayant un bord intérieur incurvé en arc de cercle avec un rayon approximativement égal à son espacement du centre de rotation autour de l'ensemble d'axe (406) et un bord extérieur incurvé en arc avec un rayon approximativement égal à son espacement du centre de rotation autour de l'ensemble d'axe (406).
